# EUROPÄISCHE PATENTSCHRIFT

(11) **EP 1 648 313 B1**
(45) Veröffentlichungstag und Bekanntmachung des Hinweises auf die Patenterteilung: **17.06.2009**
(21) Anmeldenummer: 04740942.0
(22) Anmeldetag: 13.07.2004
(51) Int. Cl.: A61B 17/128, A61B 17/122

(54) **VORRICHTUNG ZUR ENDOSKOPISCHEN APPLIZIERUNG VON SICH SCHLIESSENDEN MEDIZINISCHEN CLIPS**
DEVICE FOR THE ENDOSCOPIC APPLICATION OF SELF-CLOSING MEDICAL CLIPS
DISPOSITIF DE MISE EN PLACE ENDOSCOPIQUE DE CLIPS MEDICAUX SE REFERMANT AUTOMATIQUEMENT

(30) Priorität: 26.07.2003 DE 10334083
(43) Veröffentlichungstag der Anmeldung: 26.04.2006
(73) Patentinhaber: CARL STAHL GMBH, 73079 Süssen (DE); Medwork Medical Products and Services GmbH, 91315 Höchstadt/Aisch (DE)
(72) Erfinder: HUMMEL, Christian, 73072 Donzdorf (DE); EMBERGER, Gerhard, 73079 Süssen (DE); BAUER, Rudolf, 91352 Hallerndorf (DE)
(74) Vertreter: Bartels, Martin Erich Arthur
(86) Internationale Anmeldenummer: PCT/EP2004/007694
(87) Internationale Veröffentlichungsnummer: WO 2005/009254

(56) Entgegenhaltungen:
- DE-A- 10 203 956
- US-A- 4 416 266
- US-A- 4 671 282
- US-A1- 2002 138 083

## Beschreibung

Die Erfindung bezieht sich auf eine Vorrichtung zur endoskopischen Applizierung von sich schließenden medizinischen Clips, insbesondere für die Stillung innerer Blutungen, bei dem ein Katheterschlauch mit seinem distalen Ende im Körper eines zu behandelnden Lebewesens plaziert wird. Außerdem betrifft die Erfindung einen Clip für den Einsatz bei einer solchen Vorrichtung.

Für das Stillen innerer Blutungen, beispielsweise im oberen oder unteren Gastrointestinaltrakt, etwa von Varizenblutungen, Blutungen nach Polypektomie oder Ulcusblutungen, mit Hilfe von flexiblen Endoskopen sind verschiedene Verfahren bekannt. Als Beispiele seien Koagulation, Sklerosierung, Ligatur oder die Applizierung selbstschließender Clips genannt. Sämtliche der bekannten Verfahren sind in verschiedener Hinsicht mit Nachteilen behaftet, sei es dass der therapeutische Erfolg nicht zufriedenstellend ist oder die Anwendung aufwendig oder sehr teuer ist. Als relativ günstig, d.h. mit weniger Nachteilen behaftet, hat sich in jüngerer Zeit das Clip-Verfahren als bevorzugtes Verfahren etabliert. Weitere Anwendungen solcher Clips liegen im Bereich des Anbringens von Markierungen zur Identifizierung von Diagnostik- und Röntgenbereichen sowie von operativen Behandlungsverfahren, beispielsweise dem Entfernen von Polypen im Magen-Darmtrakt.

Beim Clip-Verfahren wird ein metallischer Clip aus dem distalen Ende eines Katheterschlauches heraus geschoben, geöffnet und an der Blutungsstelle so plaziert, dass er eine die Blutung abklemmende Klammer bildet. In dem US-Patent 5,174,276 ist eine zur Durchführung eines solchen Clip-Verfahrens vorgesehene Vorrichtung gezeigt.

Bei Durchführung des Clip-Verfahrens werden in vielen Fällen mehrere Clips zur Blutstillung benötigt. Dies führt bei den bekannten Clip-Verfahren in nachteiliger Weise zu Verzögerungen des Behandlungsablaufes weil für mehrfaches Setzen von Clips das Applikatorgerät jeweils aus dem Körper herausgenommen werden, mit einem weiteren Clip versehen und mit dem Katheterschlauch erneut in den betreffenden Körper eingeführt und an der Behandlungsstelle plaziert werden muß. Dies führt nicht nur zu einer Verlängerung des Behandlungsablaufes, was bei starken Blutungen zu einer schwerwiegenden Gefährdung des zu Behandelnden führen kann, sondern birgt auch die Gefahr von Fehlplazierungen.

Durch die DE 102 03 956 A ist eine Vorrichtung mit sich schließenden, medizinischen Clips zur endoskopischen Applizierung im Körper eines Lebewesens, insbesondere für die Stillung innerer Blutungen, bekannt, mit einem Katheterschlauch mit einem im Körper zu plazierenden distalen Ende und einem einer Bedienereinrichtung zum Betätigen der Vorrichtung zugeordneten proximalen Ende, einer sich im Katheterschlauch von der Bedienereinrichtung bis zum Bereich des distalen Endes erstreckenden Betätigungseinrichtung für das Öffnen der Schenkel eines zu applizierenden Clips, welche ein im Katheterschlauch längsverschiebbares, von der Bedienereinrichtung her steuerbares Betätigungsglied sowie ein dessen Betätigungskraft in eine Öffnungsbewegung der Schenkel des Clips umsetzendes Steuerteil aufweist, wobei das Betätigungsglied unmittelbar am Clip angreift, wobei das Steuerteil am distalen Endrand eines dem Clip zugehörigen hülsenförmigen Aufnahmeteils vorgesehen ist, in das der Clip auf einem Teil seiner Länge durch das Betätigungsglied einfahrbar ist, und wobei bei zumindest einer Ausführungsform der bekannten Lösung (Fig. 18C) durch Anlaufen der Schenkel am Steuerteil beim Einfahren des Clips in den hülsenartigen Aufnahmeteil die Schenkel zunächst geöffnet werden.

Bei einer weiteren Ausführungsform (Fig.21A - D) der bekannten Lösung ist für eine Vielzahl an Clips aufgenommen im Katheterschlauch ein gemeinsames Betätigungsglied vorgesehen, das in der Art eines einzelnen Zugseils ausgebildet in Hintereinanderfolge die Ansteuerung des jeweils zuvorderst angeordneten und zu setzenden Clips vornimmt. Die eingesetzten Clips sind von ihrer Querschnittsform her gesehen durchgehend C-förmig ausgebildet oder weisen eine sog. Alpha-Schleifenform auf, bei der sich die Schenkel des Clips kreuzen. Eine gegenseitige Abstützung der jeweiligen Schenkel eines Clips ist bei der bekannten Lösung nicht vorgesehen.

Ausgehend von diesem Stand der Technik liegt der Erfindung die Aufgabe zugrunde, eine Vorrichtung mit sich schließenden, medizinischen Clips nebst einem dahingehenden einzelnen Clip zu schaffen, die eine Abstützung der Clip-Schenkel ermöglichen und entsprechend leicht zu fertigen sind. Eine dahingehende Aufgabe löst eine Vorrichtung mit der Gesamtheit der Merkmale des Patentanspruches 1 sowie ein Clip gemäß der Merkmalsausgestaltung des Patentanspruches 11.

Dadurch, dass der Katheterschlauch selbst als Magazin dient, das vor Durchführung einer betreffenden Behandlung mit mehreren Clips geladen wird, wird ein mehrfaches Applizieren blutungsstillender Clips in unmittelbarer Folge nacheinander ermöglicht, ohne das endoskopische Gerät nach jeder Applizierung jeweils aus dem Körper entfernen zu müssen. Vielmehr kann der jeweils vorderste Clip aus dem distalen Ende des Katheterschlauches heraus geschoben, mittels des an ihm angreifenden Betätigungsgliedes geöffnet und an der zu behandelnden Blutungsstelle plaziert werden. Das Betätigungsglied wird vom Clip abgelöst, so dass dieser freigegeben wird und seine sich schließenden Schenkel die blutstillende Klemmung bewirken, wonach das Betätigungsglied mit dem im Katheterschlauch nachfolgenden Clip in Wirkverbindung gebracht wird, so dass bei Bedarf unverzüglich ein nächster Clip appliziert werden kann. Vorzugsweise werden die Clips bereits herstellerseitig geladen; es besteht insbesondere im Falle von Sonderanwendungen aber auch die Möglichkeit, vor Ort, also am Ort der Operation, die Magazinierung mit den Clips vorzunehmen. Als Katheterschlauch kommen vorzugsweise flexible zug- und druckstabile Spiralschläuche zur Anwendung, aber auch tubusartige flexible Applizierungskörper.

Bei der erfindungsgemäßen Vorrichtung befindet sich das Steuerteil, welches die Betätigungskraft des von der Bedienereinrichtung der Vorrichtung her steuerbaren Betätigungsgliedes in die Öffnungsbewegung der Schenkel des Clip umsetzt, am distalen Ende eines hülsenförmigen Aufnahmeteiles, das dem betreffenden Clip zugehörig ist. Dieser ist an seinen nebeneinander liegenden Schenkeln so geformt, dass beim Einfahren des Clip in den Aufnahmeteil durch Anlaufen je einer eine Auswölbung bildenden Knickstelle beider Schenkel am Steuerteil eine Öffnungsbewegung der freien Schenkelenden erfolgt. Wird nach dem Öffnen des erfindungsgemäßen Clip die Knickstelle an den Schenkeln mittels des Betätigungsgliedes außer Eingriff mit den hülsenförmigen Aufnahmeteil gebracht, sind die Schenkel des Clip wieder freigegeben, um sich durch ihre eigene Elastizität an der Applikationsstelle zu schließen. Dieser Schließvorgang kann durch eine Deformierung an der jeweiligen Knickstelle des Clip bedingt durch das hülsenförmige Aufnahmeteil noch weiter begünstigt und in bestimmten Ausgestaltungen auch ausschließlich durch die genannte Deformierung ersetzt werden.

Als Betätigungsglied kann ein Zugglied und als Steuerteil eine am Endrand des hülsenartigen Aufnahmeteiles befindliche, schräge Steuerfläche vorgesehen sein. Das Öffnen und anschließende Schließen der Schenkel des zu applizierenden Clip wird hierbei durch Einziehen des Clip in seinen Aufnahmeteil bewirkt, wobei durch Anlaufen der Knickstelle der Schenkel an der Steuerfläche die Öffnungsbewegung erfolgt. Durch darauffolgendes Hindurchziehen der Knickstellen durch den Aufnahmeteil wird bewirkt, dass die Clipschenkel für die Schließbewegung freigegeben werden, sobald die Knickstellen der Schenkel den hülsenförmigen Aufnahmeteil durchlaufen haben. Die schräge Steuerfläche kann auch durch einen konvexen oder konkaven Bahnverlauf einer gekrümmten Steuerfläche gebildet sein.

Als Zugglied kann ein Zugseil vorgesehen sein, und jeder Clip kann für seine Verbindung mit dem Zugseil an seinem die Schenkel verbindenden hinteren Endsteg zwei nebeneinander liegende Durchgangslöcher aufweisen, durch die das Zugseil in einer Schlaufe so hindurch geführt ist, dass sich dieses in einem vorlaufenden Strang zum Clip und von diesem in einem rücklaufenden Strang zur Bedienereinrichtung zurück erstreckt. Wenn hierbei der zwischen den Durchgangslöchern gelegene Bereich des Endsteges des Clip als Sollbruchstelle ausgebildet ist, die durch die über die Schlaufe einwirkende Zugkraft des Zugseils durchreißbar ist, kann das Zugseil auf einfache Weise nach Beendigung des Betätigungsvorganges vom Clip abgelöst werden.

Die Vorgänge des Einziehens des Clip in den Aufnahmeteil und des Ablösens des Zugseiles vom applizierten Clip gestalten sich besonders sicher, d. h. ohne die Gefahr einer Lageveränderung des Clip durch die vom Zugseil an der Sollbruchstelle ausgeübte Kraft, wenn am distalen Ende des Katheterschlauches ein den Durchtritt des hülsenartigen Aufnahmeteiles mit zugehörigem Clip nur in Austrittsrichtung nach vorn gestattendes, den hülsenartigen Aufnahmeteil jedoch gegen eine durch die Zugkraft des Zugseils bewirkte Bewegung abstützendes Sperrelement vorgesehen ist.

In besonders vorteilhafter Weise können derartige Ausführungsbeispiele in der Weise weiter ausgestaltet werden, dass im Katheterschlauch mehrere Clips mit zugehörigem, hülsenartigen Aufnahmeteil in Aufeinanderfolge angeordnet sind und dass das Zugseil mit vorlaufendem Strang und mit rücklaufendem Strang jeweils durch das eine bzw. durch das andere Durchgangsloch der Endstege sämtlicher Clips geführt ist.

Bei solchem Aufbau der Vorrichtung können mehrere Clips in unmittelbarer Aufeinanderfolge appliziert werden, weil die Vorrichtung nach dem Abreißen des Zugseiles von einem applizierten Clip durch Ziehen des Zugseiles unmittelbar in Wirkverbindung mit dem jeweils nächstfolgenden Clip gebracht wird, indem das Zugseil am Endsteg des nächstfolgenden Clip selbsttätig eine Zugschlaufe bildet. Die Vorrichtung ist somit, ohne dass irgend eine weitere Maßnahme erforderlich wäre, unmittelbar für das Applizieren eines nachfolgenden Clip bereit.

Nachstehend ist die Erfindung anhand eines in der Zeichnung dargestellten Ausführungsbeispieles im einzelnen erläutert. Es zeigen:
- Fig. 1 einen schematisch vereinfacht und gegenüber der natürlichen Größe in vergrößertem Maßstab gezeichneten Längsschnitt nur des distalen Endabschnittes eines Katheterschlauches als Bestandteil eines Ausführungsbeispieles der erfindungsgemäßen Vorrichtung;
- Fig. 2 eine perspektivisch und in noch größerem Maßstab gezeichnete Schrägansicht eines sich schließenden medizinischen Clip zur Verwendung bei der erfindungsgemäßen Vorrichtung;
- Fig. 2a eine noch weiter vergrößert gezeichnete perspektivische Darstellung nur des mit einer Sollbruchstelle versehenen Endsteges des Clip von Fig. 2;
- Fig. 3 und 4 Funktionsskizzen, lediglich zur Verdeutlichung von Bewegungen und Kräften für das Öffnen des Clips;
- Fig. 5 bis 13 Schemaskizzen nur des distalen (vorderen) Endes des in Fig. 1 gezeigten Katheterschlauches mit ausgetretenem Clip mit zugehörigem hülsenartigen Aufnahmeteil, wobei aufeinanderfolgende Betriebszustände beim Ablauf des Öffnungs- und Schließzyklusses des Clips gezeigt sind, und
- Fig. 14 eine stark vergrößerte gezeichnete perspektivische Ansicht eines in Art einer Spannzange ausgebildeten Sperrelementes für das distale Ende des Katheterschlauches.

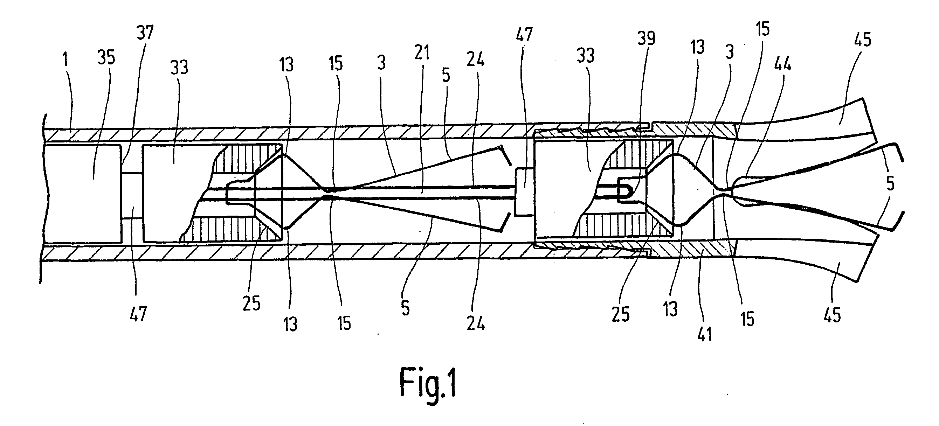

Fig. 1 zeigt den distalen Endabschnitt eines Katheterschlauches 1 als Bestandteil des zu beschreibenden Ausführungsbeispieles der erfindungsgemäßen Vorrichtung. Der Katheterschlauch 1 erstreckt sich durch den zugehörigen Arbeitsraum eines flexiblen Endoskops, das von in der Medizintechnik üblicher Bauart sein kann und mindestens einen weiteren inneren Arbeitsraum für eine Endoskopoptik einschließlich Beleuchtung und/oder andere Zwecke (beispielsweise Absaugung) enthält. Das nicht gezeigte proximale Ende des Katheterschlauches 1 ist mit der am betreffenden Ende des Endoskops befindlichen Handhabungs- und Bedienereinrichtung in Funktionsverbindung. Entsprechend der lichten Weite der Arbeitsräume bei flexiblen Endoskopen beträgt der Außendurchmesser des Katheterschlauches 1 2,7 mm.

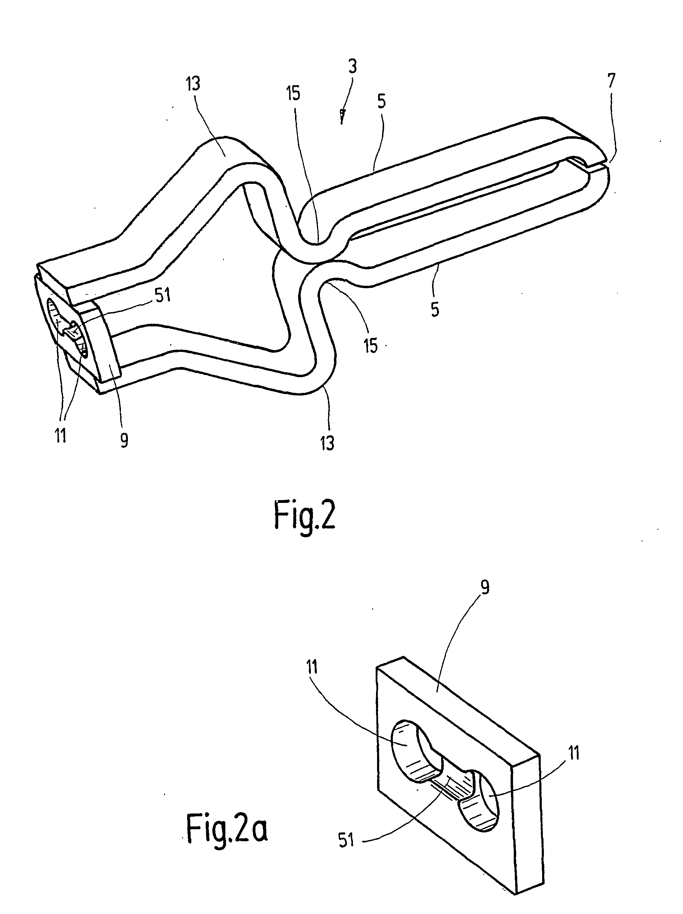

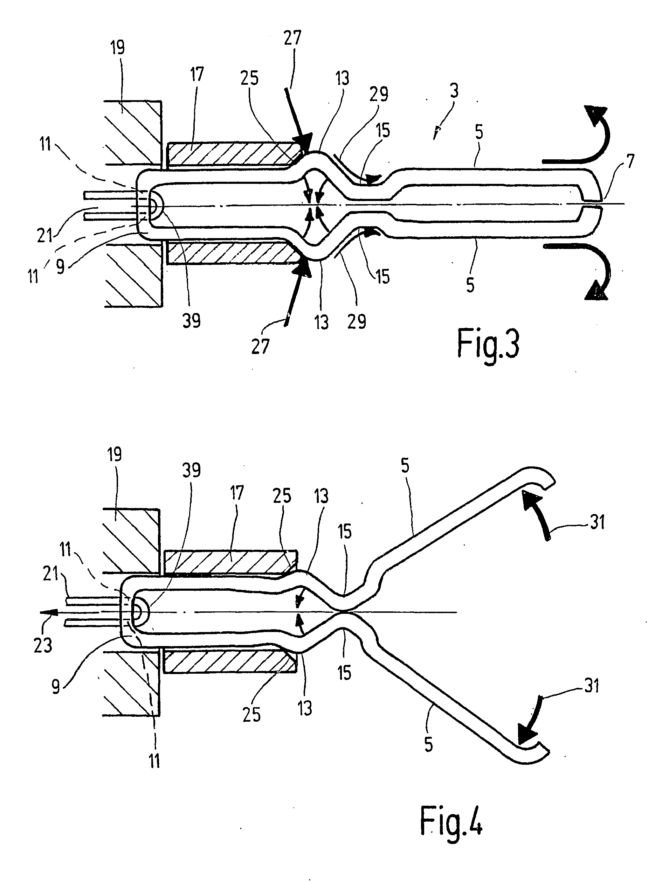

Die erfindungsgemäße Vorrichtung eignet sich zur Applizierung von sich schließenden medizinischen Clips einer Bauform, wie sie am deutlichsten aus Fig. 2 bis 4 entnehmbar ist. Wie zu ersehen ist, weist der als Ganzes mit 3 bezeichnete Clip, der aus einem für medizinische Zwecke üblichen Werkstoff, wie Edelstahl, gebildet ist, zwei Schenkel 5 auf, die, ohne sich zu überkreuzen, nebeneinander liegen und am freien Schenkelende 7 zueinander hin umgebogen sind. An dem dem Schenkelende 7 entgegengesetzten Ende sind die Schenkel 5 durch einen Endsteg 9 miteinander verbunden, der durch zwei nebeneinander liegende Durchgangslöcher 11 durchzogen ist. Vom Endsteg 9 gegen das freie Schenkelende 7 hin versetzt weist jeder Schenkel 5 einen nach außen ausgewölbten ersten Knick 13 und, gegen das Schenkelende 7 weiter versetzt, einen zweiten Knick 15 nach innen auf, welch letztere zusammen eine Abstützstelle für die gegenseitige Anlage der Schenkel 5 bilden. Vom zweiten Knick 15 erstrecken sich die restlichen Abschnitte der Schenkel 5 in in etwa parallelem Verlauf zum freien Schenkelende 7 hin, wenn sich der Clip 3 im geschlossenen Zustand befindet, wie er in Fig. 2 und 3 dargestellt ist, die im übrigen den Ausgangszustand des Clip 3 betreffen.

Die Funktionsskizzen von Fig. 3 und 4 zeigen anhand einer Hülse 17 und eines Gegenlagers 19, die das Öffnen der Schenkel 5 bewirkenden Kräfte bei Einwirkung einer mittels Zugseil 21 aufgebrachten Zugkraft auf den Clip 3 entsprechend dem Wirkungspfeil 23 von Fig. 4. Beim Anlaufen an eine schräge Steuerfläche 25 am Endrand der Hülse 17 wirkt am ersten Knick 13 eine nach einwärts gerichtete Kraft, siehe Pfeile 27, wobei die Schenkel 5 unter Streckung (siehe Pfeile 29 von Fig. 3) mit den zweiten Knicks 15 zur gegenseitigen Abstützung gelangen. Durch weiterhin wirkende Zugkraft (Pfeil 23) wälzen sich die Schenkel 5 an der Abstützstelle der zweiten Knicks 15 aneinander ab, wodurch sich die Öffnungs-Schwenkbewegung der Schenkel 5 entsprechend der Pfeile 31 (Fig. 4) ergibt.

Fig. 1 zeigt, dass im Katheterschlauch 1 mehrere Clips 3 hintereinander angeordnet sind, die jeweils mit einem hülsenförmigen Aufnahmeteil 33 eine Einheit bilden. Der Aufnahmeteil 33 entspricht in seiner Funktion in Zusammenwirkung seiner vorderen Steuerfläche 25 mit dem jeweils zugehörigen Clip 3 der Hülse 17, wie sie in Fig. 3 und 4 zur Verdeutlichung der am Clip 3 wirkenden Kräfte eingezeichnet ist. Bei der Darstellung von Fig. 1 sind zwei Clips 3 mit zugehörigem Aufnahmeteil 33 im Katheterschlauch 1 untergebracht. Bei der praktischen Anwendung kann jedoch der Katheterschlauch 1 als Magazin für 2 bis 10 Clips, vorzugsweise für 2 bis 5 Clips oder mehr vorgesehen sein. Wie Fig. 1 zeigt, sind die aus Clip 3 und Aufnahmeteil 33 bestehenden Einheiten im Katheterschlauch 1 mittels eines Schieberohres 35 verschiebbar, dessen vorderer Endrand 37 einen Druckstempel zur Anlage am benachbarten Aufnahmeteil 33 bildet und dessen proximales Ende als Teil der Betätigungseinrichtung von der Bedienereinrichtung am äußeren Betätigungsende des Endoskops her handhabbar ist.

Wie ebenfalls aus Fig. 1 in Verbindung mit Fig. 2 bis 4 ersichtlich ist, erstreckt sich im Katheterschlauch 1 als Betätigungsglied das Zugseil 21 in zwei Strängen 24 durch die Durchgangslöcher 11 (siehe Fig. 2) jedes Clip 3 hindurch, wobei am zuvorderst gelegenen Clip 3 eine Schlaufe 39 am Endsteg 9 (Fig. 1) gebildet wird. Die Schlaufe 39 ist auch in den Schemaskizzen der Fig. 3 und 4 gezeigt. Die Stränge 24 des Zugseiles 21 sind im Katheterschlauch 1 durch das Schieberohr 35 hindurch als Betätigungsglied bis zur Bedienereinrichtung am äußeren Endoskopende geführt.

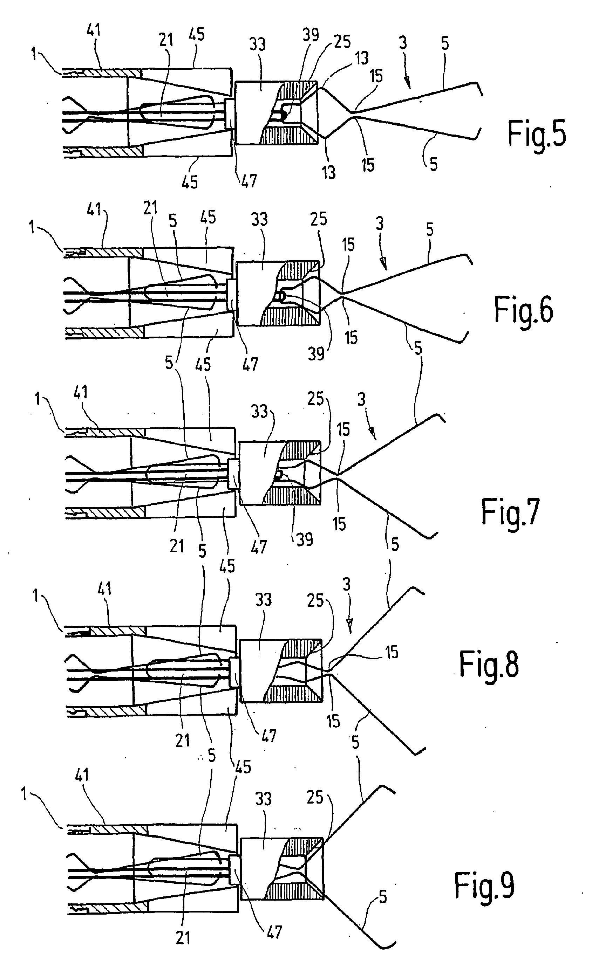

Beim Einsatz der Vorrichtung werden die im Katheterschlauch 1 hintereinander angeordneten Clips 3 mit zugehörigen Aufnahmeteilen 33 mittels des Schieberohres 35 vorgeschoben, bis der Aufnahmeteil 33 des vordersten Clip 3 den Katheterschlauch 1 verlassen hat, genauer gesagt, durch ein Sperrelement 41 hindurch getreten ist, das am distalen Ende des Katheterschlauches 1 angebracht ist. Nach dem Durchtritt durch das Sperrelement 41 befindet sich dieser Aufnahmeteil 33 mit zugehörigem Clip 3 in der in Fig. 5 gezeigten Position.

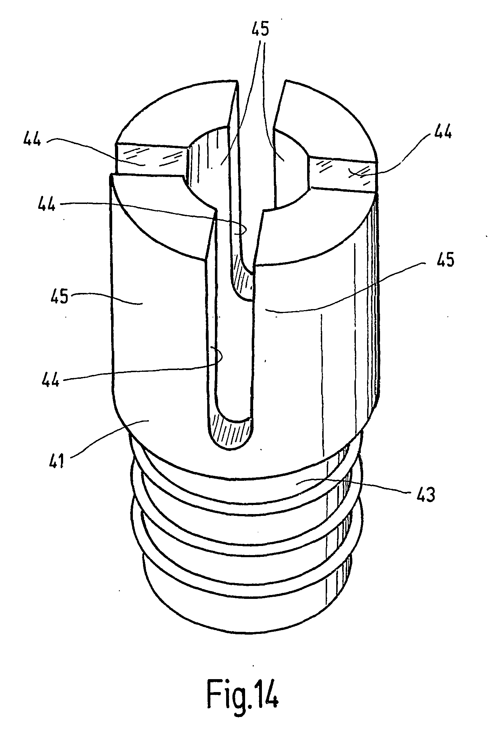

Aus Fig. 14, die das Sperrelement 41 gesondert zeigt, ist zu entnehmen, dass das Sperrelement 41 ein den Katheterschlauch 1 verlängerndes Rohrstücl< 43 ist, das in seinem Endteil Längsschlitze 44 aufweist, so dass in Art einer Spannzange Backen 45 gebildet werden, die die lichte Durchlaßweite des Rohrstückes 43 normalerweise verringern. Durch den Durchtritt eines Aufnahmeteiles 33 werden die Backen 45 elastisch leicht aufgespreizt und nehmen nach dem Austritt des Aufnahmeteiles 33 wieder die in Fig. 5 bis 13 gezeigte Sperrstellung ein, so dass der ausgetretene Aufnahmeteil 33 gegen eine Rückbewegung abgestützt ist. Ein zentraler, vorspringender Ansatz 47 des Aufnahmeteiles 33 greift dabei als Zentrierstück zwischen die Backen 45 des Sperrelementes 41 ein.

Fig. 5 bis 13 verdeutlichen in schematisch stark vereinfachter Darstellung den Ablauf des Öffnungs- und Schließzyklusses des zu applizierenden, aus dem Sperrelement 41 heraus geschobenen Clips 3 mit Aufnahmeteil 33, wobei dieser gesamte Zyklus durch Ziehen am Zugseil 21 bewirkt wird. Fig. 5 zeigt den zur Applizierung aktivierten Clip 3 mit aus dem Sperrelement 41 heraus geschobenem, hülsenartigen Aufnahmeteil 33, wobei der Clip 3 noch nicht mittels des Zugseiles 21 in den Aufnahmeteil 33 soweit eingezogen ist, dass ein Anlaufen der Knicks 13 der Schenkel 5 an der schrägen Steuerfläche 25 des Aufnahmeteiles 33 erfolgen würde. Die schräge Steuerfläche 25 entspricht der anhand der Funktionsskizzen 3 und 4 bereits besprochenen Schrägfläche 25 an der Hülse 17.

Fig. 6 bis 9 zeigen die sich durch Ziehen des Zugseiles 21 nacheinander ergebenden Betriebszustände, wobei durch Anlaufen der Knicke 13 an der Steuerfläche 25 die Schenkel 5 des Clip fortschreitend geöffnet werden, bis der Zustand der vollständigen Öffnung erreicht ist, der in Fig. 9 dargestellt.

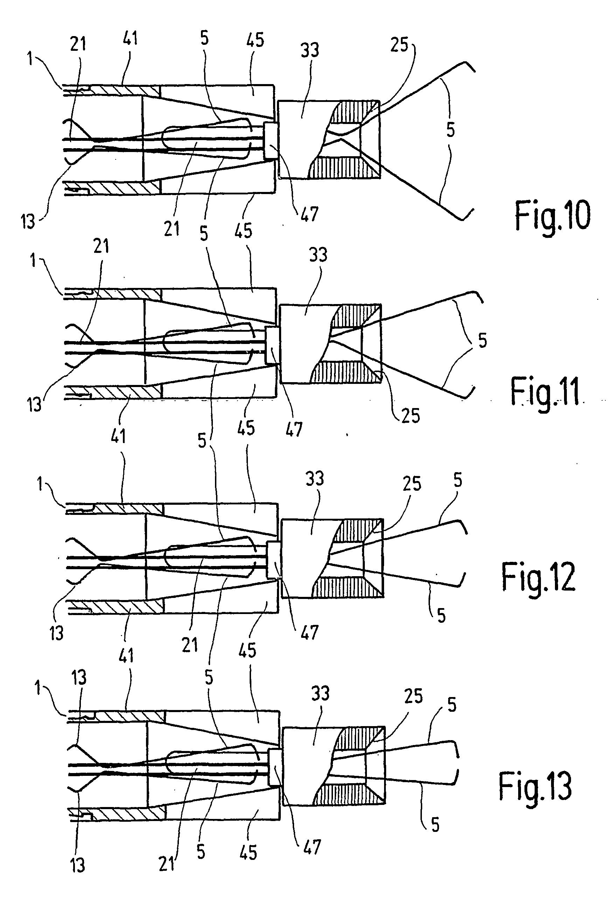

Fig. 10 bis 13 zeigen den sich bei weiterem Ziehen am Zugseil 21 ergebenden Schließzyklus, wobei die Schenkel 5 für ihre das Schließen durch die Eigenelastizität des Clip 3 bewirkte Schließbewegung und/oder seiner Deformation vollständig freigegeben sind, sobald der Clip 3 durch den Aufnahmeteil 33 entsprechend weit durchgezogen ist, dass keine Öffnungskraft mehr über die Knicke 13 ausgeübt wird. Dieser Zustand ist erreicht, wenn der Clip 3 über die in Fig. 13 gezeigte Position hinaus noch weiter durch den Aufnahmeteil 33 hindurch gezogen wird. Nach der durch das Schließen des Clip 3 erfolgten Applizierung an der zu behandelnden Blutungsstelle wird, um den applizierten Clip 3 völlig frei zu geben, am Zugseil 21 weiter gezogen, um das Zugseil von dem applizierten Clip, der sich mit seinem den Endsteg 9 (Fig. 2) aufweisenden Ende am Schenkelende 7 des im Katheterschlauch 1 nächstfolgenden Clip 3 oder am Endrand 37 des Schieberohres 35 abstützt, von diesem abzulösen. Dies geschieht, indem am Zugseil 21 stärker gezogen wird, um eine Ablösekraft aufzubringen. Vorzugsweise ist jedoch bei einer alternativen Ausführungsform vorgesehen, beim Abreißen des Zugseiles 21 den jeweiligen Clip nicht an einem nachfolgenden Clip im Magazin abzustützen, sondern vielmehr die Abstützung vorzugsweise ausschließlich über das hülsenförmige Aufnahmeteil 33 vorzunehmen. Für die dahingehende Abstützung weist der Clip zumindest teilweise quer zu seiner Öffnungsrichtung an den Schenkeln 5 zwischen dem Schenkelende 7 und dem zuordenbaren Knick 15 und diesem benachbart gegenüberliegend schneidartige Verbreiterungen (nicht dargestellt) auf, die eine Verhakungsmöglichkeit des Clip 3 im hülsenartigen Aufnahmeteil 33 ermöglichen, so dass dergestalt für das Abreißen des Zugseiles 21 über die Sollbruchstelle 51 die notwendig Gegenhaltekraft gebildet ist. Die dahingehend schneidartige Verbreiterung schließt sich flankierend beidseitig des jeweiligen Schenkels 5 des Clip 3 an.

Wie aus Fig. 2a deutlich erkennbar ist, befindet sich zu diesem Zweck in dem die Schenkel 5 des Clip 3 am hinteren Ende verbindenden Endsteg 9 zwischen den Durchgangslöchern 11 eine Sollbruchstelle 51, die so dimensioniert ist, dass durch die Schlaufe 39 des Zugseiles 21 ein Durchreißen der Sollbruchstelle 51 erfolgt, wenn eine vorbestimmte Zugkraft aufgebracht wird, die bei praktischen Ausführungsbeispielen in der Größenordnung von 40 N liegt. Der applizierte Clip 3 mit zugehörigem Aufnahmeteil 33 verbleibt somit an der Behandlungsstelle, während nach dem Abreißvorgang das Zugseil 21 selbsttätig als Schlaufe 39 am Endsteg 9 des nächstfolgenden Clip anliegt, der durch Vorschieben mittels des Schieberohres 35 für einen unmittelbar folgenden Applizierungsvorgang vorgeschoben werden kann.

Die Sollbruchstelle 51 kann gemäß der Darstellung nach der Fig.2a ausgebildet sein; es besteht aber auch die Möglichkeit, die Sollbruchstelle mit einem anders gearteten Material zu realisieren, das leicht durchreißbar ist und das verschieden ist von dem sonstigen Material des Endsteges 9. Ferner besteht auch die Möglichkeit, zur Bildung der Sollbruchstelle den Endsteg 9 homogen in seinem Material auszugestalten und es kommt zu einem Durchreißen des Endsteges 9, sofern eine vorgebbare Maximalkraft mit dem Zugseil 21 überschritten wird. Die in der Fig.14 dargestellte Spannzange ist mit ihrem Rohrstück 43 gemäß der Darstellung nach der Fig.1 am Innenumfang des freien Endes des Katheterschlauches 1 festgelegt; es besteht aber auch die Möglichkeit, dass das Rohrstück 43 das dahingehend freie Ende des Katheterschlauches 1 umfaßt und dort mittels einer Klebstoff- und/oder Preßverbindung auf Sitz gehalten ist.

Wie ersichtlich, ist der gesamte Applikationsvorgang durch Ziehen an dem als Betätigungsglied vorgesehenen Zugseil, bei dem es sich um ein feines Stahlseil handeln kann, durchführbar, also sowohl das Öffnen des jeweils aktivierten Clips 3 durch Einziehen in den Aufnahmeteil 33, das Freigeben der Schwenkbewegung der Schenkel 5 durch Durchziehen durch den Aufnahmeteil 33 und das Ablösen des Zugseiles 21 durch Abreißen der Sollbruchstelle 51, wobei das Zugseil 21 selbsttätig in Anlage an den Endsteg 9 eines im Katheterschlauch 1 nachfolgenden Clip 3 gebracht wird, so dass die Vorrichtung sofort wieder für einen nächstfolgenden Applikationsvorgang bereit ist. Mit der erfindungsgemäßen Vorrichtung ist nicht nur die Stillung von Blutungen möglich, sondern der Clip kann auch zu Markierungszwecken dienen, beispielsweise im Bereich der Diagnostik oder es sind auch operative Verfahren realisierbar, bei denen der Clip als Operationswerkzeug dient, beispielsweise zum Entfernen von Polypen im Magen-Darmtrakt oder dergleichen.

## Patentansprüche

1. Vorrichtung mit sich schließenden, medizinischen Clips (3) zur endoskopischen Applizierung im Körper eines Lebewesens, insbesondere für die Stillung innerer Blutungen, mit einem Katheterschlauch (1) mit einem im Körper zu plazierenden distalen Ende und einem einer Bedienereinrichtung zum Betätigen der Vorrichtung zugeordneten proximalen Ende, einer sich im Katheterschlauch (1) von der Bedienereinrichtung bis zum Bereich des distalen Endes erstreckenden Betätigungseinrichtung (21, 35) für das Öffnen der Schenkel (5) eines zu applizierenden Clips (3), welche ein im Katheterschlauch (1) längsverschiebbares, von der Bedienereinrichtung her steuerbares Betätigungsglied (21) sowie ein dessen Betätigungskraft in eine Öffnungsbewegung der Schenkel (5) des Clips (3) umsetzendes Steuerteil (25) aufweist, wobei das Betätigungsglied (21) unmittelbar am Clip (3) angreift, wobei das Steuerteil (25) am distalen Endrand eines dem Clip (3) zugehörigen hülsenförmigen Aufnahmeteils (33) vorgesehen ist, in das der Clip (3) auf einem Teil seiner Länge durch das Betätigungsglied (21) einfahrbar ist, **dadurch gekennzeichnet, dass** der Clip (3) an seinen beiden nebeneinanderliegenden, sich bereichsweise aneinander abstützenden Schenkeln (5) jeweils einen den Abstand der Schenkel (5) vergrößernden ersten Knick (13) nach außen und einen sich daran anschließenden, dem distalen Schenkelende (7) näher liegenden zweiten Knick (15) nach innen aufweist, der die Stelle der gegenseitigen Abstützung der Schenkel (5) bildet, so dass durch Anlaufen des ersten Knicks (13) der Schenkel (5) am Steuerteil (25) beim Einfahren des Clips (3) in den hülsenartigen Aufnahmeteil (33) die Schenkel geöffnet werden.

2. Vorrichtung nach Anspruch 1 , **dadurch gekennzeichnet, dass** die Bedienereinrichtung Bestandteil der Vorrichtung ist.

3. Vorrichtung nach Anspruch nach 1 oder 2, **dadurch gekennzeichnet, dass** als Betätigungsglied ein Zugglied (21) und als Steuerteil eine am Endrand des hülsenartigen Aufnahmeteiles (33) befindliche schräge Steuerfläche (25) vorgesehen sind.

4. Vorrichtung nach Anspruch 3, **dadurch gekennzeichnet, dass** als Zugglied ein Zugseil (21) vorgesehen ist und dass jeder Clip (3) für seine Verbindung mit dem Zugseil (21) an seinem die Schenkel (5) verbindenden hinteren Endsteg (9) zwei nebeneinanderliegende Durchgangslöcher (11) aufweist, durch die das Zugseil (21) in einer Schlaufe (39) so hindurchgeführt ist, dass sich dieses in einem vorlaufenden Strang (24) zum Clip (3) und von diesem in einem rücklaufenden Strang (24) zur Bedienereinrichtung zurück erstreckt.

5. Vorrichtung nach Anspruch 4, **dadurch gekennzeichnet, dass** der zwischen den Durchgangslöchern (11) gelegene Bereich des Endsteges (9) des Clip als Soll-Bruchstelle (51) ausgebildet ist, die durch die über die Schlaufe (39) einwirkende Zugkraft des Zugseils (21) durchreißbar ist, um das Zugseil (21) vom Clip (3) abzulösen.

6. Vorrichtung nach Anspruch 5, **dadurch gekennzeichnet, dass** am distalen Ende des Katheterschlauches (1) ein den Durchtritt des hülsenartigen Aufnahmeteils (33) mit zugehörigem Clip (3) nur in Austrittsrichtung nach vorn gestattendes, den hülsenartigen Aufnahmeteil (33) jedoch gegen eine durch die Zugkraft des Zugseils (21) bewirkte Bewegung abstützendes Sperrelement (41) vorgesehen ist.

7. Vorrichtung nach Anspruch 6, **dadurch gekennzeichnet, dass** das Sperrelement (41) ein am Ende des Katheterschlauches (1) angebrachtes Rohrstück (43) aufweist, dessen Endteil in Art einer Spannzange längsverlaufende, die Durchlaßweite normalerweise verringernde Backen (45) besitzt, die durch den hülsenartigen Aufnahmeteil (33) eines austretenden Clips (3) aus ihrer Normalstellung elastisch aufspreizbar sind und nach Rückkehr in die Normalstellung eine Abstützung des ausgetretenen Aufnahmeteils (33) gegen die Zugkraft des Zugseils (21) bilden.

8. Vorrichtung nach einem der Ansprüche 4 bis 7, **dadurch gekennzeichnet, dass** im Katheterschlauch (1) mehrere Clips (3) mit zugehörigem, hülsenartigen Aufnahmeteil (33) in Aufeinanderfolge angeordnet sind und dass das Zugseil (21) mit vorlaufendem Strang (24) und mit rücklaufendem Strang (24) jeweils durch das eine bzw. das andere Durchgangsloch (11) der Endstege (9) sämtlicher Clips (3) geführt ist.

9. Vorrichtung nach einem der Ansprüche 4 bis 8, **dadurch gekennzeichnet, dass** die Betätigungseinrichtung für den Vorschub des oder der im Katheterschlauch (1) befindlichen Clip (3) bzw. Clips (3) mit zugehörigem, hülsenartigem Aufnahmeteil (33) ein im Katheterschlauch (1) verschiebbares Rohr (35) aufweist, dessen Endrand (37) einen Druckstempel zur Anlage am zugewandten hinteren Ende des Aufnahmeteils (33) des jeweils vorzuschiebenden Clips (3) bildet.

10. Vorrichtung nach einem der Ansprüche 7 bis 9, **dadurch gekennzeichnet, dass** das der schrägen Steuerfläche (25) entgegengesetzte hintere Ende des hülsenartigen Aufnahmeteils (33) einen axial vorspringenden Ansatz (47) zur Zentrierung im spannzangenartigen Endteil des Sperrelementes (41) aufweist.

11. Clip, vorgesehen für den Einsatz bei einer Vorrichtung nach einem der Ansprüche 1 bis 10, mit zwei nebeneinander liegenden Schenkeln (5) **dadurch gekennzeichnet, dass** die Schenkel sich bereichsweise aneinander abstützen und der Clip (3) an seinen beiden Schenkeln (5) jeweils einen den Abstand der Schenkel (5) vergrößernden ersten Knick (13) nach außen und einen sich daran anschließenden, dem distalen Schenkelende (7) näherliegenden zweiten Knick (15) nach innen aufweist, der die Stelle der gegenseitigen Abstützung der Schenkel (5) bildet.

## Claims

1. An apparatus with self-closing medical clips (3) for endoscopic application in the body of a living being, in particular for arresting internal bleeding, with a catheter tube (1) with a distal end to be positioned in the body and a proximal end assigned to a user device for actuating the device, an actuation device (21, 35) extending within the catheter tube (1) from the user device to the region of the distal end for opening the legs (5) of a clip (3) to be applied which has an actuation element (21) moveable longitudinally within the catheter tube (1) and controllable by the user device and a control part (25) converting the actuation force of said actuation element into an opening movement of the legs (5) of the clip, the actuation element (21) engaging directly on the clip (3), the control part (25) being provided on the distal end edge of a bush-type retaining part (33) associated with the clip (3) into which the clip (3) can be moved over part of its length by the actuation element (21), **characterised in that** on both of its legs (5) lying next to one another and supporting one another in regions the clip (3) respectively has a first outwards kink (13) enlarging the distance between the legs (5) and a second inwards kink (15) lying closer to the distal leg end (7) which forms the point of the reciprocal support of the arms (5) so that by moving the first kink (13) of the legs (5) toward the control part (25) upon moving the clip (3) into the bush-type retaining part (33) the legs are opened.

2. The device according to Claim 1, **characterised in that** the user device is a component part of the device.

3. The device according to Claim 1 or 2, **characterised in that** as an actuation member a tension member (21) and as a control part an inclined control surface (25) located on the end edge of the bush-type retaining part (33) are provided.

4. The device according to Claim 3, **characterised in that** as a tension element a pull cord (21) is provided and that for its connection to the pull cord (21) each clip (3) has on its rear end bar (9) connecting the legs (5) two adjacent clearance holes (11) through which the pull cord (21) is passed in a loop (39) such that the latter extends in a forwards running line (24) to the clip (3) and back from the latter in a backwards running line (24) to the user device.

5. The device according to Claim 4, **characterised in that** the region of the end bar (9) of the clip positioned between the clearance holes (11) is formed as a predetermined breaking point (51) which can be severed by the tensile force of the pull cord (21) acting via the loop (39) in order to release the pull cord (21) from the clip (3).

6. The device according to Claim 5, **characterised in that** at the distal end of the catheter tube (1) a blocking element (41) only allowing the passage of the bush-type retaining part (33) with the associated clip (3) forwards in the exit direction, but supporting the bush-type retaining part (33) against a movement brought about by the tensile force of the pull cord (21) is provided.

7. The device according to Claim 6, **characterised in that** the blocking element (41) has a tube section (43) attached to the end of the catheter tube (1) the end part of which, in the manner of a chuck, has longitudinally extending jaws (45) normally reducing the passage width which can be elastically spread by the bush-type retaining part (33) of a clip (3) coming away from its normal position and after returning to the normal position form a support of the retaining part (33) that has come out against the tensile force of the pull cord (21).

8. The device according to any of Claims 4 to 7, **characterised in that** in the catheter tube (1) a number of clips (3) with an associated, bush-type retaining part (33) are arranged in sequence and that the pull cord (21) with the forwards running line (24) and with the backwards running line (24) is respectively guided through one or the other passage hole (11) of the end bars (9) of all of the clips (3).

9. The device according to any of Claims 4 to 8, **characterised in that** the actuation device for feeding the clip (3) or clips (3) located in the catheter tube (1) with the associated, bush-type retaining part (33) has a tube (35) moveable within the catheter tube (1) the end edge (37) of which forms a plunger for resting on the facing rear end of the retaining part (33) of the respective clip (3) to be moved forwards.

10. The device according to any of Claims 7 to 9, **characterised in that** the rear end of the bush-type retaining part (33) opposite the inclined control surface (25) has an axially projecting appendage (47) for centring in the chuck-type end part of the blocking element (41).

11. A clip, provided for use with a device according to any of Claims 1 to 10, with two legs (5) lying adjacent to one another, **characterised in that** the legs support one another in regions and on its two legs the clip (3) respectively has a first outward kink (13) enlarging the distance between the legs (5) and adjoining this a second inward kink (15) lying closer to the distal leg end (7) which forms the point of the mutual support of the legs (5).

## Revendications

1. Système ayant des agrafes (3) médicales se fermant pour l'application endoscopique dans le corps d'un être vivant, notamment pour arrêter des hémorragies internes, comprenant un tube (1) souple formant cathéter ayant une extrémité distale à placer dans le corps et une extrémité proximale associée à un dispositif d'opérateur pour actionner le système, un dispositif (21, 35) d'actionnement qui s'étend dans le tube (1) souple formant cathéter du dispositif de l'opérateur jusqu'à la partie de l'extrémité distale et qui est destiné à l'ouverture des branches (5) d'une agrafe (3) à appliquer, laquelle a un élément (21) d'actionnement pouvant coulisser longitudinalement dans le tube (1) souple formant cathéter et pouvant être commandé à partir du dispositif de l'opérateur, ainsi qu'une partie (25) de commande transformant sa force d'actionnement en un mouvement d'ouverture des branches (5) de l'agrafe (3), l'élément (21) d'actionnement attaquant directement l'agrafe (3), la partie (25) de commande étant prévue à l'extrémité distale d'une partie (3) de réception en forme de douille associée à l'agrafe (3), partie dans laquelle l'élément (21) d'actionnement peut faire rentrer l'agrafe (3) sur une partie de la longueur de celle-ci, **caractérisé en ce que** l'agrafe (3) a, sur ses deux branches (5) côte à côte et s'appuyant l'une contre l'autre par endroit, respectivement un premier coude (13) agrandissant la distance entre les branches (5) vers l'extérieur et un deuxième coude (15) vers l'intérieur s'y raccordant et proche de l'extrémité (7) distale des branches, qui forme le point d'appui mutuel des branches (5) de sorte que, en faisant passer le premier coude (13) des branches (5) sur la partie (25) de commande lors de l'introduction de l'agrafe (3) dans la partie (33) de réception en forme de douille, les branches s'ouvrent.

2. Système suivant la revendication 1, **caractérisé en ce que** le dispositif de l'opérateur fait partie du système.

3. Système suivant la revendication 1 ou 2, **caractérisé en ce qu'**il est prévu, comme élément d'actionnement, un élément (21) de traction et, comme partie de commande, une surface (25) de commande inclinée se trouvant sur le bord d'extrémité de la partie (23) de réception en forme de douille.

4. Système suivant la revendication 3, **caractérisé en ce qu'**il est prévu, comme élément de traction, un câble (21) de traction et **en ce que** chaque agrafe (3) a, pour sa liaison avec le câble (21) de traction à sa réglette (9) d'extrémité arrière reliant les branches (5), deux trous (11) traversants côte à côte, dans lesquels le câble (21) de traction passe en une boucle (39) de sorte que celui-ci s'étend en un brin (24), allant vers l'avant vers l'agrafe (3), et de celle-ci en un brin (24), allant vers l'arrière vers le dispositif de l'opérateur.

5. Système suivant la revendication 4, **caractérisé en ce que** la partie de la réglette (9) d'extrémité de l'agrafe mise entre les trous (11) traversant est constituée en point (51) destiné à se rompre, qui peut être déchiré par la force de traction du câble (21) de traction agissant par la boucle (39), pour détacher le câble (21) de traction de l'agrafe (3).

6. Système suivant la revendication 5, **caractérisé en ce qu'**il est prévu, à l'extrémité distale du tube (1) souple formant cathéter, un élément (41) de blocage autorisant le passage de la partie (33) de réception en forme de douille avec l'agrafe (3) associée seulement dans le sens de la sortie, mais empêchant le déplacement de la partie (33) de réception en forme de douille de se déplacer sous l'effet de la force de traction du câble (21) de traction.

7. Système suivant la revendication 6, **caractérisé en ce que** l'élément (41) de blocage a une pièce (43) tubulaire montée à l'extrémité du tube (1) souple formant cathéter et dont la partie d'extrémité a, à la manière d'une pince de serrage, des mâchoires (45), s'étendant longitudinalement et rétrécissant normalement la largeur du passage, qui peuvent être écartées élastiquement de leur position normale par la partie (33) de réception en forme de douille d'une agrafe (3) sortante et qui, après retour à la position normale, forment un appui de la partie (33) de réception sortie à l'encontre de la force de traction du câble (21) de traction.

8. Système suivant l'une des revendications 4 à 7, **caractérisé en ce que** dans le tube (1) souple formant cathéter sont disposées en succession plusieurs agrafes (3) ayant une partie (33) de réception associée en forme de douille et **en ce que** le câble (21) de traction passe par le brin (24) allant vers l'avant et par le brin (24) allant vers l'arrière respectivement dans l'un ou l'autre trou (11) de passage de la réglette (9) d'extrémité de toutes les agrafes (3).

9. Système suivant l'une des revendications 4 à 8, **caractérisé en ce que** le dispositif d'actionnement a, pour l'avance de la ou des agrafes (3) se trouvant dans le tube (1) souple formant cathéter et ayant une partie (33) de réception associée en forme de douille, un tube (53) pouvant coulisser dans le tube (1) souple formant cathéter et dont le bord (37) d'extrémité forme un poinçon de poussée pour s'appliquer à l'extrémité arrière tournée vers lui de la partie (33) de réception de l'agrafe (3) respective qui va vers l'avant.

10. Système suivant l'une des revendications 7 à 9, **caractérisé en ce que** l'extrémité arrière, opposée à la surface (25) inclinée de commande, de la partie (33) de réception en forme de douille, a un prolongement (47) faisant saillie axialement pour le centrage dans la partie d'extrémité de l'élément (41) de blocage, qui est du type à pince de serrage.

11. Agrafe prévue pour être utilisée dans un système suivant l'une des revendications 1 à 10 et ayant deux branches (5) côte à côte, **caractérisée en ce que** les branches s'appuient l'une sur l'autre par endroit et l'agrafe (3) a sur ses deux branches (5) respectivement un premier coude (13) vers l'extérieur agrandissant la distance entre les branches (5) et un deuxième coude (17) vers l'intérieur s'y raccordant et proche de l'extrémité (7) distale des branches, qui forme le point de l'appui mutuel des branches (5).
